(19) Europäisches Patentamt
European Patent Office
Office européen des brevets

(11) **EP 3 537 149 B1**

(12) **EUROPÄISCHE PATENTSCHRIFT**

(45) Veröffentlichungstag und Bekanntmachung des Hinweises auf die Patenterteilung:
**20.01.2021 Patentblatt 2021/03**

(51) Int Cl.:
***G01N 33/28*** *(2006.01)*          ***F16N 29/04*** *(2006.01)*
***G01K 13/02*** *(2021.01)*

(21) Anmeldenummer: **19160816.5**

(22) Anmeldetag: **05.03.2019**

(54) **ANTRIEB, SCHIENENFAHRZEUG UND VERFAHREN ZUM VERBESSERN DES SCHMIERMITTELEINSATZES IN EINEM ANTRIEB EINES FAHRZEUGS**

DRIVE, RAILWAY VEHICLE AND METHOD FOR IMPROVING LUBRICATION IN A DRIVE OF A VEHICLE

ENTRAÎNEMENT, VÉHICULE FERROVIAIRE ET PROCÉDÉ D'AMÉLIORATION DE L'UTILISATION DE LUBRIFIANT DANS UN ENTRAÎNEMENT D'UN VÉHICULE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.03.2018 DE 102018203258**

(43) Veröffentlichungstag der Anmeldung:
**11.09.2019 Patentblatt 2019/37**

(73) Patentinhaber: **Bombardier Transportation GmbH 10785 Berlin (DE)**

(72) Erfinder:
• **Cepak, Werner**
 **1150 Wien (AT)**
• **Bazant, Martin**
 **8708 Männedorf (CH)**
• **Dr. Vogelsberger, Markus**
 **6152 Trins (AT)**

(74) Vertreter: **Patentanwälte Bressel und Partner mbB Potsdamer Platz 10 10785 Berlin (DE)**

(56) Entgegenhaltungen:
EP-A1- 2 685 133          DE-A1-102007 021 524
DE-A1-102015 113 306     US-A1- 2017 138 876
US-B2- 9 739 763

• **Nsk: "Lagermontage und Wartung", , 1. Dezember 2010 (2010-12-01), XP055612484, Gefunden im Internet: URL:https://www.tmh-at.de/downloads/files/ de_lagermontage_und_wartung_final.pdf [gefunden am 2019-08-13]**
• **LOGAN M R ET AL: "Mechanisms and Control of Viscosity Increase in Railroad Diesel Engine Lubricants", JOURNAL OF THE AMERICAN SOCIETY OF LUBRICATION ENGINEERS,, 1. Juni 1986 (1986-06-01), Seiten 350-356, XP001337399,**

EP 3 537 149 B1

**Beschreibung**

Die Erfindung betrifft einen Antrieb, ein Schienenfahrzeug und ein Verfahren zum Verbessern des Schmiermitteleinsatzes in einem Antrieb eines Fahrzeugs und insbesondere in einem Antrieb eines Schienenfahrzeugs. Insbesondere betrifft die Erfindung das Abschätzen eines noch verfügbaren und/oder bereits verbrauchten Anteils der zulässigen Gebrauchsdauer eines Schmiermittels in einem solchen Antrieb.

Schmiermittel (oder auch Schmierstoffe) kommen zum Beispiel in Form von Öl oder Fett in verschiedenen Bereichen eines Fahrzeugantriebs zum Einsatz. Typische Einsatzorte sind fettgeschmierte oder ölgeschmierte Lager und Verzahnungen. Es ist bekannt, dass derartige Schmiermittel eine temperatur- bzw. wärmeabhängige zulässige Gebrauchsdauer besitzen, die im Folgenden auch als thermische Lebensdauer bezeichnet wird. Anders ausgedrückt ist die zulässige Einsatz- oder Gebrauchsdauer eines Schmiermittels in Anbetracht der Temperaturen, welchen das Schmiermittel ausgesetzt wird, und/oder eines Wärmeeintrags in das Schmiermittel begrenzt.

Beispielsweise nimmt die Schmierfähigkeit von Schmiermitteln ab, wenn diese über signifikante Zeiträume hohen Temperaturen ausgesetzt werden. Werte für zulässige Temperaturbereiche und/oder zulässige Zeitdauern mit vergleichsweise hohen Temperaturen (d. h. das mögliche Einsatzspektrum eines Schmiermittels) hängen dabei von den individuellen Eigenschaften eines Schmiermittels ab. Sie werden üblicherweise von dem Hersteller eines Schmiermittels angegeben oder sind aus Literaturquellen bekannt.

Im Rahmen von Wartungsarbeiten werden Schmiermittel aufgrund ihrer begrenzten Lebens- bzw. Gebrauchsdauern nach vorbestimmten Intervallen ausgetauscht. Die Intervalle werden dabei typischerweise auf Basis von Erfahrungswerten gewählt und können zum Beispiel das Alter des Schmiermittels, eine Betriebsdauer des Antriebs oder eine mittels des Antriebs bereitgestellte Fahrleistung (zum Beispiel eine Fahrdistanz) als wesentliche Kriterien heranziehen. Oftmals sind die Intervalle aber bewusst kurz definiert, um mit einer ausreichenden Sicherheit einen rechtzeitigen Austausch des Schmiermittels gewährleisten zu können, bevor dieses seine zulässige Gebrauchsdauer überschreitet.

In der Praxis führt dies aber dazu, dass die Schmiermittel oftmals aus technischer Sicht unnötig früh ausgetauscht werden, d. h. deutlich vor einem Überschreiten von deren eigentlich zulässigen Gebrauchsdauer. Über die Gesamtbetriebsdauer bzw. Lebensdauer eines Antriebs oder Fahrzeugs betrachtet kann dies dazu führen, dass der Schmiermittelverbrauch deshalb unnötig hoch ausfällt. Zusätzlich kann hierdurch der Umfang an Wartungsarbeiten über die Gesamtbetriebsdauer erhöht werden, was zu entsprechenden Kosten und Ausfallzeiten des Fahrzeugs führt.

Zusätzlich besteht aufgrund des Heranziehens von Erfahrungswerten ein Restrisiko, dass ein unerwartet frühes Überschreiten der zulässigen Gebrauchsdauer eines Schmiermittels (zum Beispiel aufgrund unerwartet hoher Belastungen) nicht erkannt wird. In einem solchen Fall können Komponenten des Antriebs beschädigt werden und erhebliche Sicherheitsrisiken entstehen.

Die DE 10 2015 113 306 A1 offenbart eine Rechenvorrichtung, die eingerichtet ist, ein Schmieröl aus einer Windkraftanlage zu überwachen, insbesondere hinsichtlich einer verbleibenden Lebensdauer. Die DE 10 2007 021 524 A1 offenbart das Auswählen eines Schmieröls für ein Getriebe, das eine minimale Viskosität für eine gegebene Betriebstemperatur aufweist. Die US 2017/138876 A1 offenbart ein Sensorsystem für einen Traktionsmotor, das einen Öltemperatursensor umfasst. Die US 9,739,763 B2 lehrt ein Flüssigkeitsanalysesystem, das Messungen hinsichtlich der Schmiermittelqualität temperaturabhängig ausführt.

Es besteht somit ein Bedarf dafür, den Einsatz von Schmiermittel in einem Antrieb eines Fahrzeugs zu verbessern, insbesondere hinsichtlich der genannten Kriterien des Schmiermittelverbrauchs, des notwendigen Umfangs von Wartungsarbeiten oder dem Vermeiden eines Überschreitens der zulässigen Gebrauchsdauer des Schmiermittels.

Diese Aufgabe wird durch ein Verfahren, einen Antrieb und ein Schienenfahrzeug gemäß den beigefügten unabhängigen Ansprüchen gelöst. Vorteilhafte Weiterbildungen sind in den abhängigen Ansprüchen angegeben. Ferner versteht es sich, dass die in der einleitenden Beschreibung erwähnten Merkmale einzeln oder in beliebiger Kombination auch bei der vorliegend offenbarten Lösung vorgesehen sein können, sofern nicht anders angegeben oder ersichtlich.

Die Erfinder haben allgemein erkannt, dass sich der Einsatz von Schmiermittel in einem Antrieb eines Fahrzeugs dadurch verbessern lässt, dass Informationen bezüglich des Zustands des Schmiermittels ermittelt werden. Genauer gesagt wird vorgeschlagen, eine Schmiermitteltemperatur zumindest mittelbar zu ermitteln und daraus auf den Zustand des Schmiermittels zu schließen, insbesondere darauf, ob dieses gegebenenfalls ausgetauscht werden muss, zum Beispiel da es ein Ende seiner zulässigen Gebrauchsdauer erreicht oder überschritten hat.

Im Detail wird ein Verfahren zum Verbessern des Schmiermitteleinsatzes in einem Antrieb eines Fahrzeugs vorgeschlagen, wobei der Antrieb einen elektrischen Traktionsmotor umfasst und wenigstens einen Bereich mit Schmiermittel, und wobei das Verfahren umfasst:

- Ermitteln des Werts von wenigstens einer Betriebsgröße des Fahrzeugs;

- Ermitteln einer Schmiermitteltemperatur (T) basierend auf dem Wert der Betriebsgröße; und

- Abschätzen einer noch verfügbaren Gebrauchsdauer des Schmiermittels und/oder eines bereits verbrauchten Anteils der zulässigen Gebrauchsdauer des Schmiermittels basierend auf der Schmiermitteltemperatur (T);

wobei die Betriebsgröße wenigstens eine thermische Verlustleistung des Antriebs (30) ist.

Unter dem Antrieb eines Fahrzeugs kann eine Anordnung verstanden werden, die eine Antriebskraft erzeugt und hierdurch Räder oder wenigstens einen Radsatz des Fahrzeugs antreibt, sodass sich die Räder oder der Radsatz daraufhin unter Erzeugen einer Vorschubkraft an der Umgebung (zum Beispiel einer Schiene) abstützen können. Der Antrieb kann allgemein auch als Antriebsstrang bezeichnet werden.

Bei dem Fahrzeug kann es sich um ein Schienenfahrzeug handeln und insbesondere um eine Lokomotive oder einen Triebwagen.

Das angestrebte Verbessern des Schmiermitteleinsatzes kann sich beispielsweise auf eine der vorstehend genannten Kriterien oder, anders ausgedrückt, Verbesserungspotentiale in Form eines Verringerns des Umfangs von Wartungsarbeiten, einem Reduzieren des Schmiermittelverbrauchs oder dem Vermeiden eines Überschreitens der zulässigen Schmiermittelgebrauchsdauer beziehen.

Der elektrische Traktionsmotor kann auch als Antriebsmotor oder Antriebsmaschine bezeichnet werden. Er kann allgemein dazu dienen, wenigstens ein Rad oder wenigstens einen Radsatz des Fahrzeugs anzutreiben und insbesondere zu rotieren. Hierdurch kann in der bereits geschilderten Weise eine Vorschubkraft (bzw. Traktionskraft) zur Fortbewegung des Fahrzeugs erzeugt werden. Bei dem Traktionsmotor kann es sich z. B. um einen Drehstromasynchronmotor handeln oder um einen Synchronmotor, insbesondere in Form eines permanentmagneterregten Motors.

Bevorzugt wird ein Drehstromasynchronmotor mit einem Kurzschlussläufer verwendet. In an sich bekannter Weise kann der elektrische Traktionsmotor (oder genauer gesagt eine Ausgangswelle hiervon) mit einem Getriebe des Antriebs gekoppelt sein. Das Getriebe kann eine Radsatzwelle des Fahrzeugs antreiben, die drehmomentübertragend und vorzugsweise verdrehgesichert mit einem Radsatz gekoppelt ist. Insbesondere kann die Radsatzwelle die einzelnen Räder des Radsatzes miteinander verbinden. In dem Getriebe kann wenigstens eine Verzahnung vorhanden sein, mit der beispielsweise eine veränderliche oder nicht veränderliche Übersetzung und bevorzugt eine Untersetzung bereitgestellt werden kann. Die Verzahnung kann über wenigstens zwei ineinandergreifende Zahnräder des Getriebes bereitgestellt werden.

Der Antrieb kann ferner über wenigstens ein Lager oder eine Lagerstelle verfügen, welche insbesondere als ein Wälzlager realisiert sein können. Das Lager oder die Lagerstelle können eine Komponente des Antriebs lagern und insbesondere drehbar lagern. Ein solches Lager kann z.B. in oder an dem Traktionsmotor bereitgestellt sein, beispielsweise um eine Ausgangswelle hiervon (insbesondere eine Läuferwelle) und/oder den Rotor zu lagern. Zusätzlich oder alternativ kann das Lager in oder an einem Getriebe des Antriebs bereitgestellt sein, z.B. um etwaige Zahnräder hiervon drehbar lagern zu können. Bei dem Lager oder der Lagerstelle kann es sich allgemein um ein mittels Schmiermittel geschmiertes Lager handeln.

Der Antrieb umfasst ferner wenigstens einen Bereich, in dem ein Schmiermittel vorhanden ist, wobei der Bereich wenigstens eine Komponente (und insbesondere eine schmiermittelgelagerte oder schmiermittelgeführte Komponente) des Antriebs umfassen kann. Beispielsweise kann der Bereich ein durch das Schmiermittel geschmiertes Lager umfassen oder ein Getriebe mit einem Ölbad und/oder einer geschmierten Verzahnung.

Das Ermitteln der Schmiermitteltemperatur (z. B. in Form eines Schmiermitteltemperaturwerts oder -temperaturverlaufs) kann durch ein direktes Erfassen der Schmiermitteltemperatur erfolgen (z.B. durch einen Kontakt zwischen einem Temperatursensor und dem Schmiermittel). Alternativ kann das Ermitteln der Schmiermitteltemperatur mittelbar erfolgen (beispielsweise durch eine Temperaturmessung in einem Bereich, dessen Wärmeübertragung an ein bestimmtes Schmiermittelvolumen bekannt oder berechenbar ist).

Beispielsweise kann die Temperaturmessung an einem Außenring eines Lagers erfolgen, das in seinem Innern ein Schmiermittelvolumen umfasst, und die Temperatur des Schmiermittels kann anhand einer Wärmeübertragung von dem Außenring auf das Schmiermittelvolumen berechnet oder abgeschätzt werden. Die Wärmeübertragung kann in herkömmlicher Weise unter Berücksichtigung einer Geometrie (z.B. einer Fläche und/oder einer Dicke eines Materialvolumens, über das eine Wärmeleitung erfolgt), einer Wärmeleitfähigkeit und/oder eines vorherrschenden Temperaturunterschiedes berechnet werden. Für sämtliche der geschilderten Berechnungen können Simulationsprogramme verwendet werden, wie sie auf dem Markt erhältlich sind.

Das Abschätzen der Gebrauchsdauer des Schmiermittels kann umfassen, dass eine bereits verbrauchte thermische Lebensdauer (bzw. ein verbrauchter Anteil der zulässigen Gebrauchsdauer) und/oder eine noch verbleibende thermische Lebensdauer des Schmiermittels (bzw. ein noch verbleibender Anteil der zulässigen Gebrauchsdauer) bestimmt oder abgeschätzt wird. Insbesondere kann die noch verbleibende Gebrauchsdauer als Absolutwert abgeschätzt werden und zum Beispiel in einer Einheit von noch verbleibenden Zeitstunden angegeben werden.

Unter der thermischen Lebensdauer bzw. zulässigen Gebrauchsdauer kann die zulässige Einsatzdauer des Schmiermittels verstanden werden, welche in Folge von thermischen Belastungen, denen das Schmiermittel ausgesetzt ist, mit zunehmender Betriebsdauer abnehmen kann. Folglich kann das Abschätzen der verbleibenden oder verbrauchten zulässigen Gebrauchsdauer Informationen darüber liefern, wie lange das Schmiermittel noch verwendet werden kann (z.B. in Bezug auf eine noch verbleibende mögliche Betriebsdauer oder verbleibende zurücklegbare Fahrdistanz des Antriebs bzw. des Fahrzeugs). Ebenso kann das Abschätzen der verbleibenden oder verbrauchten zulässigen Gebrauchsdauer Informationen darüber liefern, welcher Anteil an der maximalen Einsatzdauer des Schmiermittels bereits aufgebraucht ist.

Das Abschätzen der zulässigen Gebrauchsdauer kann auf Basis hinterlegter Zusammenhänge zu der ermittelten Schmiermitteltemperatur erfolgen. Beispielsweise können in einer Ermittlungseinrichtung des Antriebs oder des Fahrzeugs Zusammenhänge dahingehend hinterlegt werden, mit welchem aufgebrauchten und/oder noch verbleibenden Anteil der zulässigen Gebrauchsdauer ein über ein bestimmtes Zeitintervall ermittelter mittlerer oder maximaler Schmiermitteltemperaturwert einhergeht. Derartige Zusammenhänge können z.B. in Form einer Datenbank oder einer Tabelle hinterlegt sein (z.B. in Form einer sogenannten Lookup-Tabelle) und vorab berechnet werden (d. h. vor einem realen Betrieb des Antriebs, der außerhalb von Versuchsreihen stattfindet).

Derartige Zusammenhänge kann auch in Form eines Diagramms hinterlegt sein oder als eine Funktion, welche ein entsprechendes Diagramm beschreibt. Lebensdauerdiagramme für Schmiermittel sind z.B. im Kontext von Wälzlagern bekannt. In diesem Fall wird ein sogenannter Temperaturfaktor Kt als Temperaturkorrekturfaktor auf der einen Achse und eine Lagertemperatur auf der anderen Achse des Diagramms eingetragen. Mit dem Temperaturfaktor Kt lässt sich allgemein eine noch verbleibende Lebensdauer des in dem Wälzlager verwendeten Schmiermittels Bestimmen. Hierfür kann der Temperaturfaktor Kt mit weiteren optionalen Korrekturfaktoren sowie mit einer sogenannten Grundfettgebrauchsdauer tf multipliziert werden, um die verfügbare Schmiermittellebensdauer in Anbetracht einer vorliegenden Temperatur zu berechnen. Für den gegebenen Anwendungsfall kann ein analoges Diagramm bereitgestellt oder als funktionaler Zusammenhang hinterlegt werden, sodass von der ermittelten Schmiermitteltemperatur auf dessen verbleibende Gebrauchsdauer geschlossen werden kann.

Alternativ kann das Abschätzen auf Basis einer Berechnung der zulässigen Gebrauchsdauer erfolgen, wobei die ermittelte Schmiermitteltemperatur als eine Ausgangs- bzw. Eingangsgröße verwendet wird. Anders ausgedrückt kann der verbrauchte Anteil und/oder die noch verbleibende zulässige Gebrauchsdauer des Schmiermittels als eine Funktion der ermittelten Schmiermitteltemperatur berechnet werden. Als ermittelte Schmiermitteltemperatur kann dabei wiederum eine über ein bestimmtes Zeitintervall (und/oder eine zurückgelegte Fahrdistanz) ermittelte mittlere oder maximale Schmiermitteltemperatur betrachtet werden können.

Für das Berechnen kann allgemein von einem logarithmischen Zusammenhang zwischen der ermittelten Schmiermitteltemperatur und der thermischen Lebensdauer des Schmiermittels ausgegangen werden. Gemäß einer Variante kann von folgender Formel ausgegangen werden:

$$t = B^{(a*(Tmax-T))} \quad (1).$$

Dabei bezeichnen die einzelnen Variablen Folgendes:

t [h] : verbleibende Gebrauchsdauer;
Tmax [°C] : theoretischer Temperaturmaximalwert, bei dem sich eine Lebensdauer von einer Stunde ergibt;
T [°C] : aktuelle Schmiermitteltemperatur (oder auch gemittelte oder maximale Schmiermitteltemperatur über ein aktuelles Zeitintervall);
B [h] : schmiermittelspezifischer (d.h. schmiermitteltyp- oder produktspezifischer) Indikator für die zulässige Gebrauchsdauer des Schmiermittels;
a [1/°C] : schmiermittelspezifischer (d.h. schmiermitteltyp- oder produktspezifischer) Indikator für die Temperaturabhängigkeit der Gebrauchsdauer des Schmiermittels.

Die Formel bringt demnach allgemein zum Ausdruck, welche Gebrauchsdauer (bzw. thermische Lebensdauer) in Anbetracht einer aktuell ermittelten Schmiermitteltemperatur verbleibt. Die Variablen B und a sind dabei im Wesentlichen oder ausschließlich von Materialeigenschaften des verwendeten Schmiermittels abhängig. Sie können aus der Literatur ermittelt werden oder sind durch Herstellerangaben bekannt.

Als Orientierung kann davon ausgegangen werden, dass eine Erhöhung der Schmiermitteltemperatur um einen Betrag von 10 Kelvin bis 15 Kelvin typischerweise zu einer Halbierung von dessen Gebrauchsdauer führt.

Basierend auf der vorstehenden Formel (1) kann auch ermittelt werden, welcher Anteil der insgesamt verfügbaren zulässigen Gebrauchsdauer in einem betrachteten Zeitintervall verbraucht wurde. Für ein definiertes Zeitintervall Δt [h] kann der verbrauchte Anteil an der insgesamt zulässigen bzw. verfügbaren Gebrauchsdauer ΔL [%] wie folgt berechnet werden:

$$\Delta L = \frac{\Delta t}{B^{(a*(Tmax-T))}} \quad (2).$$

Die dabei eingesetzte Schmiermitteltemperatur kann einen mittleren oder maximalen Temperaturwert in dem betrachteten Zeitintervall betreffen. Die einzelnen Werte für die Anteile der verbrauchten zulässigen Gebrauchsdauer ($\Delta L$) können auch für mehrere betrachtete Zeitintervalle oder allgemein fortlaufend aufsummiert werden. Hierüber kann ein kumulierter Wert bezüglich des bereits verbrauchten Anteils der insgesamt verfügbaren zulässigen Gebrauchsdauer ermittelt werden. Zieht man diesen kumulierten Anteil, welcher wiederum in der Einheit % vorliegt, von einem Wert von 100 % ab, erhält man den noch verbleibenden Anteil an der insgesamt verfügbaren zulässigen Gebrauchsdauer. Dieser Anteil liegt ebenfalls in der Einheit % vor.

Es versteht sich, dass in Kenntnis einer ursprünglich verfügbaren maximal zulässigen Gebrauchsdauer, die in einer Einheit von Stunden angegeben ist, aus den vorstehend ermittelten prozentualen Anteilen ferner die noch verfügbare Gebrauchsdauer oder bereits verbrauchte Gebrauchsdauer jeweils in der Einheit von Stunden ermitteln lässt.

Aus den gewonnenen Informationen bezüglich der bereits verbrauchten oder noch verbleibenden zulässige Gebrauchsdauer kann auch abgeschätzt werden, wann voraussichtlich ein Austausch des Schmiermittels erforderlich werden wird. Anders ausgedrückt kann der Zeitpunkt der Wartung abgeschätzt werden, in deren Rahmen ein Schmiermittelaustausch erfolgen muss. Dies kann jedoch voraussetzen, dass die Betriebsbedingungen und insbesondere die dabei auftretenden Schmiermitteltemperaturen nicht signifikant von den bisherigen Temperaturen abweichen.

Zusätzlich oder alternativ kann das Abschätzen der verbleibenden oder verbrauchten Gebrauchsdauer dazu verwendet werden, sicherzustellen, dass das Schmiermittel in jedem Fall vor einem Erreichen oder Überschreiten von dessen zulässiger Gebrauchsdauer ausgetauscht wird. Beträgt der z.B. gemäß obiger Formel berechnete verbrauchte Anteil der zulässigen Gebrauchsdauer 90 %, 95 % oder 100 % (bzw. der noch verbleibende Anteil der zulässigen Gebrauchsdauer 10 %, 5 % oder 0 %) kann eine Warnung ausgegeben werden, welche auf die Notwendigkeit eines Austauschens des Schmiermittels hinweist.

Ein Vorteil des Verfahrens besteht somit zunächst darin, dass basierend auf tatsächlich beobachteten Betriebsbedingungen in Form der ermittelten Schmiermitteltemperatur auf einen Zustand des Schmiermittels geschlossen werden kann. Statt also lediglich anhand von Erfahrungswerten über einen etwaigen Zustand des Schmiermittels und insbesondere einen verbrauchten oder noch verbleibenden Anteil von dessen zulässiger Gebrauchsdauer zu spekulieren, kann mit einer erhöhten Zuverlässigkeit auf dessen tatsächlichen Zustand in Form der bereits verbrauchten oder noch verbleibenden zulässigen Gebrauchsdauer geschlossen werden. Somit besteht eine größere Gewissheit dahingehend, ob ein Austausch des Schmiermittels tatsächlich erforderlich ist oder wann ein solcher Austausch voraussichtlich erforderlich werden wird. Ein unnötig verfrühter Austausch ebenso aber ein unerwünschtes Überschreiten der zulässigen Gebrauchsdauer können somit verhindert werden. Über die Gesamtbetriebsdauer des Fahrzeugs betrachtet kann hierdurch die Anzahl an insgesamt erforderlichen Wartungsvorgängen reduziert werden (z.B. da unnötig frühe Wartungen samt Austausch des Schmiermittels vermieden werden). Ebenso kann der über die Gesamtbetriebsdauer betrachtete Schmiermittelverbrauch reduziert werden, da ein unnötig häufiges Austauschen des Schmiermittels vermieden werden kann. Mit anderen Worten ermöglicht das hierin vorgestellte Verfahren somit, die zulässige Gebrauchsdauer des Schmiermittels auszureizen und insbesondere möglichst vollständig auszureizen, ohne aber die Betriebssicherheit unnötig zu gefährden, da das Erreichen oder Überschreiten der zulässigen Gebrauchsdauer rechtzeitig erkannt und angezeigt werden kann.

In einer Weiterbildung des Verfahrens und des Antriebs ist die Schmiermitteltemperatur auf Basis eines gemessenen Temperaturwerts ermittelbar, insbesondere wobei der Temperaturwert in dem oder nahe des Bereichs gemessen wird, in dem das Schmiermittel vorhanden ist. Wie geschildert, kann die Schmiermitteltemperatur unmittelbar bzw. direkt gemessen werden oder aus einem an anderer Stelle gemessenen Temperaturwert mittelbar berechnet werden.

Diese Variante ermöglicht allgemein, dass die Schmiermitteltemperatur besonders realitäts- bzw. einsatznah bestimmt wird und darauf basierend ermittelte Informationen bezüglich der Gebrauchsdauer eine hohe Aussagekraft besitzen.

Erfindungsgemäß ist ferner Folgendes vorgesehen:

- Ermitteln des Werts wenigstens einer Betriebsgröße des Fahrzeugs; und
- Ermitteln der Schmiermitteltemperatur basierend auf dem Wert der Betriebsgröße.

Insbesondere kann die Schmiermitteltemperatur basierend auf den ermittelten Werten verschiedener Betriebsgrößen des Fahrzeugs ermittelt werden. Die Schmiermitteltemperatur kann daher auf Basis einer Kombination verschiedener Betriebsgrößen ermittelt werden. In dieser Beschreibung werden noch Betriebsgrößen genannt. Je nach Ausgestaltung des Verfahrens kann die Schmiermitteltemperatur auf Basis einer beliebigen Kombination dieser Betriebsgrößen ermittelt werden.

Bei der Betriebsgröße kann es sich um eine Größe handeln, welche einen Betriebszustand des Fahrzeugs definiert und/oder nach Maßgabe eines aktuellen Betriebszustands des Fahrzeugs unterschiedliche Werte annehmen kann. Die Betriebsgröße kann in einer Ermittlungseinrichtung des Antriebs- oder des Fahrzeugs bereitgestellt sein oder durch diese ermittelt werden. Insbesondere kann die Betriebsgröße in Form von Daten innerhalb einer solchen Ermittlungseinrichtung vorliegen (z.B. in Form sogenannter Betriebsdaten) oder durch die Ermittlungseinrichtung ermittelbar sein. Übergeordnet kann es sich bei der Betriebsgröße um eine von der Schmiermitteltemperatur verschiedene Größe handeln.

Das Ermitteln der Schmiermitteltemperatur basierend auf dem Wert der Betriebsgröße kann umfassen, dass die Schmiermitteltemperatur basierend auf dem Betriebsgrößenwert berechnet wird. Hierzu kann beispielsweise ein funktioneller Zusammenhang zwischen der Betriebsgröße und der Schmiermitteltemperatur hinterlegt sein (z.B. in einer Ermittlungseinrichtung des Antriebs oder des Fahrzeugs). Ein solcher funktionaler Zusammenhang kann vorab versuchstechnisch erfasst werden oder im Rahmen eines Simulations- oder Wärmeübertragungsmodells ermittelt werden. Alternativ kann ein tabellarischer Zusammenhang oder, allgemein ausgedrückt, eine Zuordnung von Schmiermitteltemperaturen zu möglichen Werten der Betriebsgröße hinterlegt sein, was ebenfalls in einer Ermittlungseinrichtung des Antriebs oder des Fahrzeugs erfolgen kann.

Im Rahmen der vorliegenden Offenbarung kann allgemein vorgesehen sein, dass die Ermittlungseinrichtung in eine Steuereinrichtung des Antriebs oder des Fahrzeugs integriert oder als eine solche Steuereinrichtung realisiert ist. Dabei kann es sich z.B. um eine zentrale Steuereinrichtung des Antriebs- oder des Fahrzeugs handeln, mittels der der Betrieb des Antriebs bzw. des Fahrzeugs gesteuert wird.

Die Werte der Betriebsgröße können allgemein gemessen oder berechnet werden. Im Falle eines Berechnens der Betriebsgröße kann diese auf Basis einer anderen Betriebsgröße und vorzugsweise auf Basis einer im Betrieb gemessenen Betriebsgröße berechnet werden. Anders ausgedrückt kann die Betriebsgröße zumindest mittelbar aus einer anderen im Betrieb gemessenen Größe des Antriebs oder des Fahrzeugs ermittelt werden. Dies betrifft zum Beispiel eine nachfolgend geschilderte Motortemperatur, die anhand anderer Betriebsgrößen abgeschätzt oder berechnet wird.

Bei der Betriebsgröße kann es sich um eine Zugkraft handeln, die von dem Antrieb (oder dem Fahrzeug) aufgebracht wird. Unter der Zugkraft kann allgemein diejenige Kraft verstanden werden, welche der Antrieb bzw. das Fahrzeug zum Erzeugen einer Fortbewegung aufbringen und insbesondere auf mit dem Antrieb oder Fahrzeug gekoppelte weitere Einheiten ausüben kann (z.B. auf etwaige mit dem Fahrzeug gekoppelte Anhänger oder Waggons). Hierbei kann es sich insbesondere um denjenigen Anteil einer von dem Antrieb erzeugten Traktionskraft handeln, der in Anbetracht eines Schlupfes zwischen dem angetriebenen Rad oder Radsatz und einem Untergrund (z.B. eine Schiene) für eine tatsächliche Fortbewegung bzw. einen tatsächlichen Vorschub verbleibt.

Die Zugkraft kann z.B. auf Basis einer vorgegebenen Fahrgeschwindigkeit und/oder Fahrleistung (zum Beispiel einem Antriebsdrehmoment) berechnet werden. Werte bezüglich eines vorherrschenden Schlupfes können dabei ebenso berücksichtigt und auf Basis von Schätzungen oder Erfahrungswerten ermittelt werden.

Weiter kann die Betriebsgröße eine Geschwindigkeit des Antriebs und/oder des Fahrzeugs betreffen. Beispielsweise kann eine Geschwindigkeitsgröße des Traktionsmotors in Form einer Drehzahl oder Drehgeschwindigkeit betrachtet werden. Ebenso kann eine Fahrgeschwindigkeit des Fahrzeugs betrachtet werden. In bekannter Weise kann eine Geschwindigkeitsgröße des Traktionsmotors in eine Fahrgeschwindigkeit (und umgekehrt) umgerechnet werden, zumindest wenn ein Schlupf zwischen den Fahrzeugrädern und dem Untergrund (zum Beispiel einer Schiene) vernachlässigbar ist oder mit einer ausreichenden Sicherheit geschätzt werden kann. Die Umrechnung erfordert dabei in bekannter Weise Kenntnisse über den Antrieb bzw. Antriebsstrang, mittels dem die Traktionsmotordrehzahl bzw. -drehung in eine Raddrehung umgewandelt wird.

Die Betriebsgröße kann sich ebenso auf die Umgebungstemperatur beziehen oder diese angeben.

Ferner kann die Betriebsgröße eine der folgenden Größen sein:

- eine zum Antreiben des Traktionsmotors verwendete Stromstärke;
- eine zum Antreiben des Traktionsmotors verwendete Spannung;
- eine zum Antreiben des Traktionsmotors verwendete Erregerfrequenz.

Bei der Stromstärke und/oder der Spannung kann es sich um eine Stromstärke bzw. Spannung handeln, die eingangsseitig an dem Traktionsmotor anliegt. Insbesondere kann es sich um eine Stromstärke bzw. Spannung handeln, die von einem Stromrichter oder Spannungswandler bereitgestellt wird, welche den Traktionsmotor mit elektrischer Energie versorgt. Beispielsweise kann es sich um eine Stromstärke oder Spannung eines Wechselrichters oder Frequenzumrichters handeln, die eingangsseitig an dem Traktionsmotor anliegt. Ist der Traktionsmotor in Form eines Drehstromasynchronmotors bereitgestellt, kann es sich um eine Stromstärke und Spannung handeln, die an den Ständerwicklungen des Traktionsmotors anliegt.

Auch die Erregerfrequenz kann durch einen Stromrichter oder Spannungswandler bereitgestellt werden und eingangsseitig an dem Traktionsmotor anliegen. Insbesondere kann es sich wiederum um eine Erregerfrequenz von bzw. für Ständerwicklungen des Traktionsmotors handeln.

Erfindungsgemäß wird als Betriebsgröße eine thermische Verlustleistung innerhalb des Antriebs und insbesondere innerhalb des Traktionsmotors berücksichtigt. Zusätzlich kann wenigstens eine weitere Betriebsgröße jeglicher hierin geschilderten Art ermittelt und für eine Schmiermitteltemperatur-Ermittlung verwendet werden. Ferner kann die Betriebsgröße eine Temperatur des Traktionsmotors betreffen, insbesondere eine berechnete und/oder gemessene Temperatur des Traktionsmotors. Zum Messen der Temperatur des Traktionsmotors kann in herkömmlicher Weise eine Temperaturmessung nahe oder an den Ständerwicklungen des Traktionsmotors erfolgen. Hierzu können Temperaturfühler z.B. in Form von Widerstandstemperaturfühlern wie sogenannten Pt100-Sensoren verwendet werden.

Zum Berechnen der Temperatur des Traktionsmotors kann wenigstens eine weitere Betriebsgröße herangezogen werden und insbesondere eine Betriebsgröße der vorstehend erläuterten Art. Gemäß einer Variante des Verfahrens und des Antriebs wird die Temperatur des Traktionsmotors auf Basis wenigstens einer thermischen Verlustleistung berechnet. In diesem Zusammenhang kann auch eine Umgebungstemperatur betrachtet werden. Eine entsprechende Verlustleistung kann zum Beispiel anhand von Motordrehzahl bestimmt werden, wobei der Zusammenhang zwischen einer Drehzahl und einer Erwärmung bzw. Temperatur des Traktionsmotors experimentell oder auf Basis von Simulations- und/oder Berechnungsmodellen ermittelt werden kann.

Im Vergleich zu einem Messen der Temperatur des Traktionsmotors kann ein (insbesondere rechnerisches) Ermitteln dieses Temperaturwertes sich auf einen beliebigen Bereich des Traktionsmotors und insbesondere auf einen für die Schmiermittelerwärmung relevanten Bereich fokussieren. Wie geschildert, werden Motortemperaturen bisher in vorbestimmten Bereichen des Traktionsmotors bestimmt und sind gegebenenfalls in anderweitigen Bereichen des Motors nicht oder nur mit einem hohen messtechnischen Aufwand bestimmbar. Die auf diese Weise gemessenen Temperaturwerte können jedoch für das Ermitteln einer Schmiermitteltemperatur wenig relevant und/oder nicht mit einer ausreichenden Genauigkeit auf Temperaturwerte in anderen tatsächlich interessierenden Bereichen des Traktionsmotors umgerechnet werden. Insbesondere kann es schwierig sein, Wärmeübertragungen innerhalb des Traktionsmotors im Rahmen einer Berechnung geeignet zu modellieren oder zu berücksichtigen, was ein Verwenden eines tatsächlich gemessenen Motortemperaturwerts zur Bestimmung der Schmiermitteltemperatur erschweren oder verhindern kann.

Die Temperatur des Traktionsmotors kann auch auf Basis eines vorab hinterlegten Zusammenhangs zu wenigstens einer der anderen Betriebsgröße ermittelt werden. Der Zusammenhang kann wiederum in Form eines funktionalen Zusammenhangs hinterlegt sein, wobei die Traktionsmotortemperatur in Abhängigkeit wenigstens einer der anderen Betriebsgröße definiert ist. Ebenso kann ein Zusammenhang hinsichtlich von Werten der Betriebsgröße zu Temperaturwerten des Traktionsmotors hinterlegt sein, beispielsweise in Form einer Wertetabelle oder Datenbank. Sämtliche der geschilderten Zusammenhänge können im Rahmen von Versuchsreihen oder durch Simulation oder Berechnung ermittelt werden.

Die thermische Verlustleistung kann eine Reibungsverlustleistung an wenigstens einer Lagerstelle (bzw. einem Lager) des Antriebs betreffen, insbesondere an einer Lagerstelle in Form eines Wälzlagers. Diese Reibungsverlustleistung kann z.B. in an sich bekannter Weise aus der Lagerkraft und einer (zumindest im Falle eines Wälzlagers) Drehzahl des Lagers oder der von dem Lager gelagerten Komponente (z.B. Welle) berechnet werden.

Die Drehzahl kann in Kenntnis der Geschwindigkeit des Fahrzeugs oder des Traktionsmotors (zum Beispiel in Form von dessen Drehzahl) und der Übersetzungsverhältnisse innerhalb des Antriebs bestimmt werden. Hierfür können in bekannter Weise die geometrischen Abmessungen und Übersetzungsverhältnisse innerhalb des Antriebs betrachtet werden. Die Lagerkraft kann unter Anwenden herkömmlicher Kräfte- und Momentengleichgewichte aus den innerhalb des Antriebs vorherrschenden Kräften und Momenten ermittelt werden. Handelt es sich bei der Lagerstelle bzw. dem Wälzlager um eine Lagerstelle zum Lagern eines Getriebes (insbesondere eines Zahnrades hiervon) oder einer zumindest mittelbar hiermit gekoppelten Komponente, kann die Lagerkraft aus den vorliegenden Verzahnungskräften errechnet werden. Die Verzahnungskräfte können wiederum aus innerhalb des Antriebs vorherrschenden Kräften und Momenten bestimmt werden, z.B. einem Drehmoment des Traktionsmotors, einer erzeugten Zugkraft (die auch unmittelbar als Betriebsgröße erfasst oder berechnet werden kann), und den geometrischen Verhältnissen innerhalb des Antriebs. Die geometrischen Verhältnisse können insbesondere Radien oder Durchmesser von Wellen oder Zahnrädern des Antriebs betreffen sowie damit einhergehende Übersetzungsverhältnisse. Schließlich kann die Massenverteilung innerhalb des Antriebs zum Bestimmen der Lagerkraft herangezogen werden und insbesondere die Masse einer von der Lagerstelle gelagerten Komponente.

Die thermische Verlustleistung kann ferner in Form einer Reibungsverlustleistung in einem Getriebe des Antriebs vorliegen. Derartige Reibungsverluste können an bzw. zwischen Zahnrädern des Getriebes auftreten. Sie können in bekannter Weise in Abhängigkeit der Drehzahl und der Verzahnungskräfte bestimmt werden, wobei die Verzahnungskräfte z.B. aus den geometrischen Verhältnissen (bspw. Radien der Zahnräder, Zahnformen bzw. -winkel, Übersetzungsverhältnisse zwischen den Zahnrädern) und der Zugkraft oder dem übertragenen Drehmoment ermittelbar sind.

Weiter kann die thermische Verlustleistung wenigstens eine Eisenverlustleistung des Traktionsmotors betreffen. Eisenverlustleistungen können innerhalb des Traktionsmotors am Ständer und/oder am Rotor auftreten. Insbesondere können Eisenverlustleistungen in bekannter Weise in bzw. im Bereich von Blechpaketen des Ständers und/oder des Läufers entstehen. Genauer gesagt können die Eisenverluste auf Wirbelstromverluste und/oder Hystereseverluste in Zusammenhang mit Ummagnetisierungsvorgängen beim Betrieb des Traktionsmotors zurückzuführen sein. Sie können anhand der Stromstärke bestimmt werden, welche z.B. an den Wicklungen des Ständers anliegt. Diese Stromstärke kann in bekannter Weise in einen magnetischen Fluss bzw. eine magnetische Flussdichte umgerechnet werden, die mittels dem Ständer erzeugbar ist. Der entsprechende Fluss wie auch die Frequenz der an den Ständerwicklungen anliegenden Ströme kann zur Berechnung der Eisenverluste herangezogen werden. Ist die Frequenz der Ströme (oder allgemein die Erregerfrequenz) unbekannt, kann diese aus einer gemessenen oder vorgegebenen Drehzahl des Traktionsmotors berechnet werden (oder auch aus einer anderen Größe, die in eine entsprechende Drehzahl umrechenbar ist, wie beispielsweise die Fahrgeschwindigkeit des Fahrzeugs). Allgemein kann von einem proportionalen Zusammenhang zwischen der Erregerfrequenz und der Drehzahl ausgegangen werden.

Schließlich kann die thermische Verlustleistung (zusätzlich oder alternativ) eine Wicklungsverlustleistung des Traktionsmotors betreffen. Die Wicklungsverlustleistung kann insbesondere eine Kupferverlustleistung betreffen (beispielsweise eine Kupferverlustleistung in den Ständerwicklungen und/oder in etwaigen Rotorwicklungen). Insbesondere im Fall der Ständerwicklungen können die Kupferverluste anhand der anliegenden Stromstärke bestimmt werden. Kupferverluste in der Rotorwicklung können in an sich bekannter Weise in Abhängigkeit der Drehfeldleistung bestimmt werden. Hierfür können Betriebsgrößen wie die Motordrehzahl, ein anliegender Strom- oder eine Spannung und/oder ein aktuell erzeugtes Drehmoment des Traktionsmotors oder eine Zugkraft des Antriebs berücksichtigt werden.

Wie bereits geschildert, kann die Schmiermitteltemperatur auf Basis eines hinterlegten Zusammenhangs zu Werten der wenigstens einen Betriebsgröße ermittelt werden, insbesondere auf Basis eines hinterlegten tabellarischen Zusammenhangs. Hierfür können die Betriebsgrößenwerte bestimmt und anschließend auf Basis des hinterlegten Zusammenhangs und insbesondere in einer Tabelle oder Datenbank ermittelt werden, welcher Schmiermitteltemperaturwert mit dieser Betriebsgröße einhergeht. Der entsprechende Zusammenhang kann im Rahmen von Testfahrten mit dem Fahrzeug oder einem vergleichbaren Fahrzeug oder Antrieb ermittelt worden sein. Alternativ oder zusätzlich können Simulationen durchgeführt werden. Diese Variante ermöglicht zum einen das besonders schnelle und wenig rechenintensive bestimmen von Schmiermittel Temperaturwerten. Ferner werden hierfür keine gesonderten Temperatursensoren benötigen.

Wie ebenfalls bereits diskutiert, kann die Schmiermitteltemperatur auf Basis des Werts der Betriebsgröße berechnet werden. Hierfür können zum Beispiel die thermischen Verlustleistungen ermittelt und als Wärmequellen innerhalb des Antriebs betrachtet werden. Auf Basis herkömmlicher thermodynamischer Modellierungen des Antriebs können daraufhin Wärmeübertragungen von diesen Wärmequellen an Schmiermittelvolumina oder allgemein an Bereiche mit einem Schmiermittel innerhalb des Antriebs berechnet werden. Dies ermöglicht ein zuverlässiges Ermitteln der Schmiermitteltemperatur, ohne dass zusätzliche Sensorik zwingend erforderlich ist.

Schließlich kann die Gebrauchsdauer auf Basis einer maximalen und/oder mittleren Schmiermitteltemperatur über einen vorbestimmten Zeitraum abgeschätzt werden. Durch Berücksichtigen einer maximalen Schmiermitteltemperatur kann sichergestellt werden, dass thermische Höchstbelastungen in jedem Fall erfasst und berücksichtigt werden. Das Berücksichtigen einer mittleren Schmiermitteltemperatur ermöglicht, dass die tatsächlichen Betriebsbedingungen möglichst umfassend berücksichtigt werden und nicht nur etwaige Extremzustände hiervon.

Die Erfindung betrifft ferner einen Antrieb zum Antreiben eines Fahrzeugs, insbesondere eines Schienenfahrzeugs, mit:

- einem elektrischen Traktionsmotor;
- wenigstens einem Bereich mit einem Schmiermittel; und
- einer Ermittlungseinrichtung, die dazu eingerichtet ist, eine Schmiermitteltemperatur zu ermitteln, und darauf basierend eine noch verfügbare Gebrauchsdauer und/oder einen bereits verbrauchten Anteil der zulässigen Gebrauchsdauer des Schmiermittels abzuschätzen,
  sowie mit den weiteren Merkmalen des beigefügten Anspruchs 9.

Der Antrieb kann jegliche Weiterbildung und jegliches weitere Merkmal umfassen, um sämtliche der vorstehenden oder nachstehenden Schritte, Betriebszustände und Funktionen bereitzustellen oder auszuführen. Insbesondere kann der Antrieb dazu eingerichtet sein, ein Verfahren gemäß jeglichem der vorstehenden oder nachstehenden Aspekte auszuführen.

Schließlich betrifft die Erfindung auch ein Schienenfahrzeug, umfassend einen Antrieb der vorstehend geschilderten Art.

Im Folgenden werden Beispiele und wird eine Ausführungsform der Erfindung anhand der beigefügten schematischen Figuren erläutert. In Ihrer Art und/oder Funktion übereinstimmende Merkmale können dabei ausführungsformübergreifend mit den gleichen Bezugszeichen versehen sein.

Es stellen dar:

Fig. 1 einen erfindungsgemäßen Antrieb in Draufsicht, mittels dem ein Verfahren gemäß einem ersten Beispiel ausgeführt wird;

Fig. 2 eine Frontalansicht auf den Antrieb aus Fig. 1;

Fig. 3 ein Ablaufschema des Verfahrens gemäß dem ersten Beispiel; und

Fig. 4 ein Ablaufschema eines Verfahrens gemäß einem zweiten Beispiel.

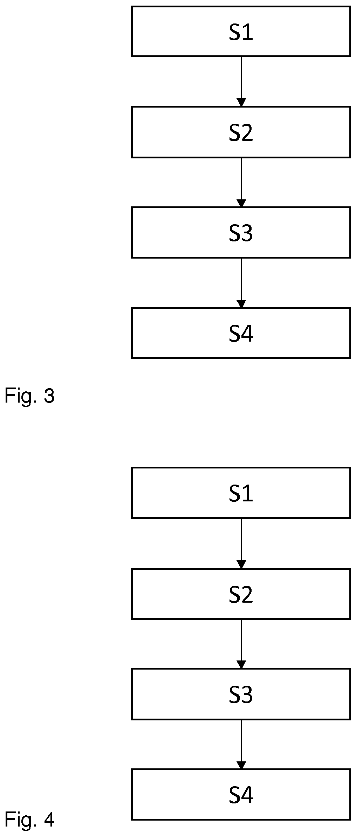

Im Folgenden werden Beispiele erläutert, wobei das Beispiel gemäß Fig. 4 einer erfindungsgemäßen Ausführungsform gemäß den beigefügten Ansprüchen entspricht. Die anderen Beispiele sind hiermit kombinierbar.

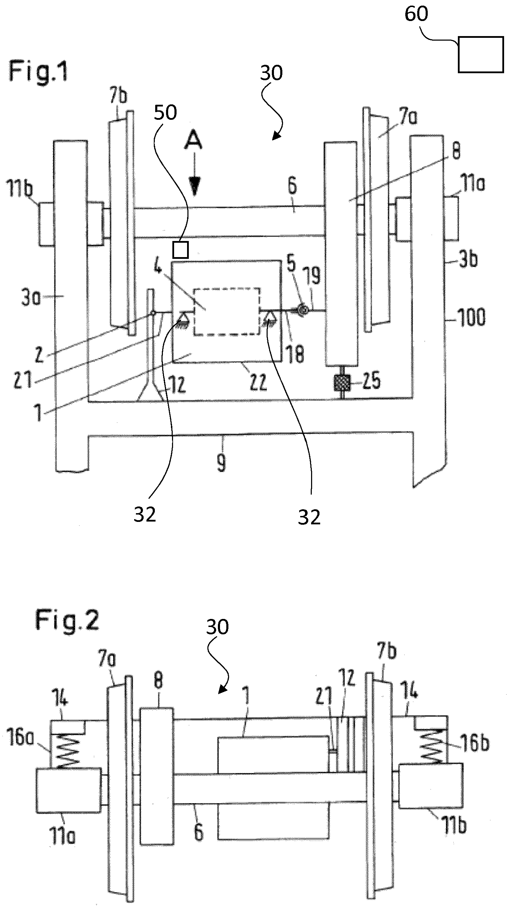

Fig. 1 zeigt eine Draufsicht auf ein Drehgestell mit einem Radsatz. Das Drehgestell ist zum Einsatz in einem Schienenfahrzeug vorgesehen und wird über einen Antrieb 30 in Form eines Querantriebs angetrieben. Das Drehgestell weist einen Drehgestellrahmen 100 mit einem beispielhaft in einer Fahrtrichtung des Schienenfahrzeugs offenen H-förmigen Tragprofil auf, dessen Querträger mit 9 bezeichnet ist und dessen Längsträger mit 3a, 3b bezeichnet sind. An gegenüberliegenden Längsträgern des Drehgestellrahmens 100 sind Lager 11a und 11b angeordnet, in denen die Radsatzwelle 6 des Radsatzes 7a, 7b drehbar gelagert ist. Die Radsatzwelle 6 wird über ein achsreitendes Getriebe 8 angetrieben, welches über eine elastische Aufhängung 25 an dem Querträger 9 aufgehängt ist. Das Antriebsmoment wird über eine Antriebswelle 19 in das Getriebe 8 eingeleitet.

Das Getriebe 8 umfasst wenigstens 2 nicht gesondert dargestellte Zahnräder, nämlich ein erstes Zahnrad, das zur gemeinsamen Rotation drehfest mit der Antriebswelle 19 gekoppelt ist, sowie ein zweites Zahnrad, dass zur gemeinsamen Rotation drehfest mit der Radsatzwelle 6 gekoppelt ist. Das erste und das zweite Zahnrad greifen dabei ineinander, um das von dem Traktionsmotor 1 bereitgestellte Drehmoment mittels eines unveränderlichen Übersetzungsverhältnisses (insbesondere in Form einer Untersetzung) auf die Radsatzwelle 6 zu übertragen. Hierdurch wird der Radsatz 7a, 7b rotiert und eine Traktionskraft auf nicht gesondert dargestellte Schienen aufgebracht, wodurch ein Vorschub bzw. eine Zugkraft erzeugt wird. Prinzipiell kann aber auch eine andere Anzahl von Zahnrädern vorgesehen sein sowie ein veränderliches Übersetzungsverhältnis durch Einlegen verschiedener Gänge in dem Getriebe 8.

Die Antriebswelle 19 ist über ein kardanisch bewegliches Gelenk 5 von der Läuferwelle 18 eines elektrischen Traktionsmotors 1 angetrieben. Das kardanisch bewegliche Gelenk 5 weist bezogen auf die Rotationsachse der Läuferwelle 18 eine axiale Nachgiebigkeit bzw. Beweglichkeit auf.

In dem gezeigten Fall ist der Traktionsmotors 1 als ein Drehstromasynchronmotor ausgebildet und wird von einem nicht dargestellten Wechselrichter mit elektrischer Energie versorgt. Genauer gesagt werden nicht gesondert dargestellte Kupferwicklungen eines Ständer 22 des Traktionsmotors 1 von dem Wechselrichter mit einer definierten Stromstärke, einer definierten Spannung und einer definierten Erregerfrequenz versorgt, woraufhin ein sich drehendes Magnetfeld zum Antreiben eines Läufers 4 des Traktionsmotors 1 erzeugt wird. Die Kupferwicklungen sind in herkömmlicher Weise auf Blechpaketen des Ständers 22 angeordnet bzw. werden hiervon getragen. Der Läufer 4 ist in dem gezeigten Fall als ein Kurzschlusskäfigläufer ausgebildet und umfasst nicht gesondert dargestellte Kupferwicklungen, die ebenfalls auf Blechpaketen angeordnet sind und hiervon getragen werden. Die jeweiligen Blechpakete sind in Figur 1 nicht gesondert dargestellt. Schließlich erkennt man, dass die Läuferwelle 18 an beidseitig von dem Läufer 4 angeordneten Lagerstellen 32 drehbar gelagert ist. Die Lagerstellen 32 werden dabei durch lediglich schematisch abgebildete Wälzlager bereitgestellt.

An dem Ständer 22 ist eine schematisch dargestellte Befestigung 21 angebracht, die über eine kardanisch bewegliche Aufhängung 2 an einer Längsstütze 12 aufgehängt ist, welche an dem Querträger 9 befestigt ist.

Der Traktionsmotor 1 sowie die Komponenten, welche ein hiervon erzeugtes Drehmoment auf die Räder des Radsatzes 7a, 7b übertragen (einschließlich eben dieses Radsatzes 7a, 7b) bilden den eigentlichen Antrieb 30 oder, mit anderen Worten, den Antriebsstrang des gezeigten Beispiels. Der Antrieb 30 umfasst demnach den Traktionsmotor 1, die Läuferwelle 18, die Antriebswelle 19, das Gelenk 5, das Getriebe 8, die Radsatzwelle 6 sowie den Radsatz 7a, 7b. Insbesondere das Gelenk 5 ist aber lediglich optional, sodass die Läuferwelle 18 auch direkt mit dem Getriebe 8 gekoppelt sein kann.

Der Antrieb 30 umfasst aber auch eine elektronische und/oder digitale Ermittlungseinrichtung 60, welche in dem gezeigten Beispiel in eine Steuereinrichtung des Schienenfahrzeugs integriert ist. Die Ermittlungseinrichtung 60 ist dazu eingerichtet, die nachstehend geschilderten Maßnahmen zum Bestimmen der verbrauchten oder noch verfügbaren Gebrauchsdauer von verwendetem Schmiermittel durchzuführen. Hierfür kann die Ermittlungseinrichtung 60 allgemein eine Rechen- oder Prozessoreinheit umfassen und bereitgestellte Eingangssignale zum Beispiel in Form von Betriebsdaten erfassen und verarbeiten.

Schließlich ist ein Temperatursensor 50 in Form eines sogenannten Pt100-Sensors schematisch angedeutet. Diese ist dazu eingerichtet, eine Temperatur der Wicklungen des Ständers 22 zu erfassen. Es handelt sich hierbei um eine bei bestehenden Systemen bereits eingesetzte Variante, bei denen der Temperatursensor 50 dazu dient, den Betrieb des Traktionsmotors 50 zu überwachen.

Fig. 2 zeigt die Anordnung in einer Frontansicht gemäß dem Pfeil A aus Fig. 1. Dabei ist zusätzlich noch eine Federung 16a, 16b zu erkennen, über die die Radlager 11a, 11b federnd mit einem Wagenkasten 14 des Schienenfahrzeugs verbunden sind, welches den Antrieb 30 umfasst.

Innerhalb des Antriebs 30 kommt in verschiedenen Bereichen Schmiermittel zum Einsatz. Dies betrifft zunächst die Lagerstellen 32 der Läuferwelle 18 des Traktionsmotors, bei denen hierfür insbesondere eine Fettschmierung oder Ölschmierung infrage kommt. Ferner sind die Zahnräder des Getriebes an ihren Zähnen mit Schmiermittel versehen und/oder Durchlaufen bei ihrer Rotation ein Ölbad. Auch das Gelenk 5 sowie die Aufhängung 2 können Schmiermittel umfassen und jeweils als geschmierte Lagerstellen des Antriebs 30 aufgefasst werden.

Im Betrieb des Antriebs 30 (d. h. wenn der Traktionsmotor 1 mit elektrischer Energie versorgt wird und gegebenenfalls auch ein Drehmoment bzw. einen Vorschub erzeugt) können sich die ein Schmiermittel umfassenden Bereiche erwärmen. Abseits einer allgemein Erwärmung durch die Umgebungstemperatur betrifft dies zum Beispiel eine Erwärmung aufgrund verschiedener thermischer Verlustleistungen innerhalb des Antriebs 30. Insbesondere im Bereich des Traktionsmotors 1 können verschiedene Verlustleistungen auftreten, welche zu einer Erwärmung der Lagerstellen 32 und dem dort vorhandenen Schmiermittel führen.

Dies betrifft zum Beispiel Eisenverluste innerhalb der Blechpakete von Ständer 22 und Läufer 4. Ebenso treten Wicklungsverluste in Form von Kupferverlusten in den Wicklungen von Ständer 22 und Läufer 4 auf. Die Lagerstellen 32 des Traktionsmotors 1 können sich auch aufgrund von Reibung bei einer Rotation der Läuferwelle 18 erwärmen (zum Beispiel Reibung innerhalb der dortigen Wälzlager). In diesem Zusammenhang sind auch Lagerkräfte an den Lagerstellen 32 relevant, welche zumindest mittelbar von den Zahnrädern des Getriebes 8 und den dort wirkenden Verzahnungskräften ausgehen (d. h. die Zahnräder des Getriebes 8 können sich bei direkter Kopplung über die Wellen 19, 18 an den Lagerstellen 32 abstützen).

In dem Getriebe 8 entstehen ebenfalls thermische Verlustleistungen aufgrund von Reibungsverlusten zwischen den dortigen Zahnrädern. Gleiches gilt für Reibungen in dem Gelenk 5 oder Aufhängung 2. Möglichkeiten zum Ermitteln der geschilderten thermischen Verlustleistungen wurden vorstehend bereits allgemein beschrieben und werden im Folgenden noch näher erläutert.

Wird das Schmiermittel in den jeweiligen Bereichen aufgrund der Verlustleistungen erwärmt, verkürzt sich der noch verfügbare Anteil der Gebrauchsdauer aufgrund verschiedener an sich bekannter chemischer Zersetzungsprozesse innerhalb des Schmiermittels. Um zu bestimmen, welche Gebrauchsdauer noch verfügbar ist und/oder bereits verbraucht wurde, sieht eine erste erfindungsgemäße Variante deshalb vor, mittels nicht gesondert dargestellter thermischer Sensoren (zum Beispiel in Form von Temperaturmessfühlern wie einem sogenannten Pt100-Sensor) Temperaturen nahe oder in wenigstens einem der geschilderten Bereiche zu erfassen, welche über ein Schmiermittel verfügen.

Derartige Sensoren können zum Beispiel innerhalb des Getriebes 8 (zum Beispiel nahe oder in Kontakt mit einem dortigen Ölbad) positioniert sein. Ebenfalls können Sie an den Lagerstellen 32 des Traktionsmotors 1 positioniert sein und zum Beispiel eine Temperatur von Außenringen der dortigen Wälzlager messen. Auch ein Messen an der Aufhängung 2 (zum Beispiel durch Anbringen an dem Längsstütze 12) oder dem Gelenk 5 (zum Beispiel durch Anbringen an einer der beiden 18, 19) ist möglich.

Über vorbestimmte Zeitintervalle von zum Beispiel mehreren Sekunden, Minuten oder Stunden wird daraufhin die gemessene Temperatur in dem jeweiligen Bereich aufgezeichnet. Dies kann zum Beispiel durch ein Übermitteln der Messsignale bzw .-daten von den Temperatursensoren an die Ermittlungseinrichtung 60 erfolgen. Die gemessene Temperatur in dem Zeitintervall kann gemittelt werden. Alternativ kann ein Maximalwert bestimmt werden. Basierend auf der eingangs geschilderten Gleichung (2) kann dann ein in diesem Zeitintervall verbrauchter Anteil an der Gebrauchsdauer des Schmiermittels für den jeweiligen Bereich bestimmt werden. Derartige Anteile können über mehrere Zeitintervalle aufsummiert werden, um einen kumulierten Wert zu erhalten und fortlaufend den verbrauchten (und/oder noch verbleibenden) Anteil der zulässigen Gebrauchsdauer des Schmiermittels zu bestimmen.

Wird das Ende der Gebrauchsdauer erreicht oder überschritten (zuzüglich eines optionalen Toleranzbereichs), kann die Ermittlungseinrichtung 60 ein Warnsignal ausgeben, zum Beispiel an einen Fahrer des Schienenfahrzeugs. Somit wird sichergestellt, dass das Schmiermittel nur dann ausgetauscht wird, wenn dies tatsächlich erforderlich ist, was den Schmiermittelverbrauch und den erforderlichen Umfang an Wartungsarbeiten reduzieren kann. Gleichzeitig ist aber gewährleistet, dass das Schmiermittel in jedem Fall rechtzeitig (d. h. ohne signifikantes Überschreiten von dessen zulässiger Gebrauchsdauer) ausgetauscht wird.

Bezugnehmend auf die Figuren 3 und 4 werden im Folgenden weitere Möglichkeiten zum Ermitteln des verbrauchten und/oder verbleibenden Anteils der Schmiermittelgebrauchsdauer für den Antrieb 30 der Figuren 1 und 2 beschrieben. In beiden der weiteren Fällen werden verschiedene Betriebsgrößen in Form von Betriebsdaten erfasst. Bildlich gesprochen werden diese Betriebsgrößen als Eingangsgrößen einer "Blackbox" betrachtet, wobei die Ausgangsgröße die Schmiermitteltemperatur ist. Die Blackbox selbst kann das (mathematische) Wärmeübertragungsmodell oder thermodynamische Modell des Antriebs 30 enthalten.

Bei den nachstehend geschilderten Varianten ist es jeweils nicht erforderlich, gesonderte Temperatursensoren an den ein Schmiermittel umfassenden Bereichen vorzusehen. Dies kann optional aber dennoch erfolgen. Ferner ist darauf hinzuweisen, dass die geschilderten Betriebsgrößen in bestehenden Systemen problemlos verfügbar sind und beispielsweise in Form entsprechender Betriebsdaten an Steuereinrichtungen des Schienenfahrzeugs ausgegeben werden. Folglich kann auch die Ermittlungseinrichtung 60 ohne besonderen Aufwand auf die entsprechen Betriebsgrößen zugreifen. Für bestimmte Betriebsgrößen kann aber auch vorgesehen sein, dass diese aus den erfassten Betriebsdaten erst ermittelt werden und nicht unmittelbar zu Verfügung stehen. Auch ein derartiges mittelbares Ermitteln von Betriebsgrößen kann von der Ermittlungseinrichtung 60 ausgeführt werden.

Anhand des Ablaufschemas von Figur 3 wird zunächst eine Variante diskutiert, bei der anhand hinterlegter Zusammenhänge und insbesondere versuchstechnisch ermittelter Zusammenhänge eine Schmiermitteltemperatur aus den Betriebsdaten ermittelt wird.

Im Schritt S1, der auch vorab vervollständigt werden kann und deshalb nur ein optionaler Schritt des eigentlichen Verfahrens ist, wird im Rahmen eines Versuchsaufbaus die Schmiermitteltemperatur in einem interessierenden Bereich gemessen (zum Beispiel an den Lagerstellen 32 des Traktionsmotors 1). Eine derartige Messung erfolgt bevorzugt im Betrieb des Antriebs 30 und zum Beispiel bei einer Testfahrt des Schienenfahrzeugs oder eines vergleichbaren Schienenfahrzeugs mit demselben Antrieb 30. Für die Messung können wiederum Temperatursensoren verwendet werden.

Neben der Schmiermitteltemperatur werden die parallel hierzu vorliegenden Werte der erfassten Betriebsgrößen aufgezeichnet. Hierbei handelt es sich es sich um die folgenden Betriebsgrößen, wobei erfindungsgemäß aber auch andere und vor allem nur ausgewählte der aufgelisteten Betriebsgrößen erfasst und aufgezeichnet werden können:

- eine Zugkraft, die von dem Antrieb aufgebracht wird (zum Beispiel mittelbar bestimmbar aus einem Drehmoment des Traktionsmotors);
- eine Drehzahl des Traktionsmotors;
- eine Fahrgeschwindigkeit des Fahrzeugs;
- eine Umgebungstemperatur;
- eine zum Antreiben des Traktionsmotors verwendete Stromstärke;
- eine zum Antreiben des Traktionsmotors verwendete Spannung;
- eine zum Antreiben des Traktionsmotors verwendete Erregerfrequenz.

Die Betriebsgrößen definieren einen konkreten Betriebspunkt des Schienenfahrzeugs 60. In der Ermittlungseinrichtung 60 werden die Werte dieser Betriebsgrößen für einen Betriebspunkt gesammelt abgespeichert (zum Beispiel als ein geschlossener Betriebspunkt-Datensatz). Dem entsprechenden Datensatz wird dann der dabei vorliegende und parallel gemessene Schmiermitteltemperaturwert zugeordnet. Folglich wird ein Zusammenhang zwischen den Betriebsgrößen bzw. einem hiervon definierten Betriebspunkt und einem dabei vorliegenden Schmiermitteltemperaturwert erfasst und in der Ermittlungseinrichtung 60 hinterlegt.

Wird das Schienenfahrzeug daraufhin zu einem späteren Zeitpunkt außerhalb der Versuchsreihe und insbesondere im eigentlichen Transportverkehr eingesetzt, können die Betriebsgrößen wiederum laufend erfasst werden. Dies ist in Figur 3 als Schritt S2 dargestellt. Daraufhin kann im Schritt S3 ein aktuell vorliegender Betriebspunkt aus den vorab (d. h. während der Versuchsreihen) hinterlegten Datensätzen ermittelt werden, der sich durch identische oder vergleichbare Werte der Betriebsgrößen auszeichnet. Für den entsprechenden Betriebspunkt kann daraufhin der dazugehörige Schmiermitteltemperaturwert im Schritt S4 durch die Ermittlungseinrichtung 60 ausgelesen werden. Dieser Schmiermitteltemperaturwert kann in identischer Weise zum vorstehend geschilderten Vorgehen bei einer gemessenen Schmiermitteltemperatur verwendet werden, um den verbrauchten und/oder noch verbleibenden Anteil der Gebrauchsdauer des Schmiermittels zu ermitteln.

Zu betonen ist, dass derartige Datensätze für ein konkretes Schienenfahrzeug vorab hinterlegt werden können (d. h. vor einer Auslieferung oder dem Beginn eines eigentlichen Transportbetriebs). Hierfür kann auch auf Ergebnisse oder Datensätze von Versuchsreihen zurückgegriffen werden, welcher mittels anderer aber vorzugsweise baugleicher Schienenfahrzeuge gewonnen wurden. Ferner können spätestens nach dem Ermitteln der Datensätze durch Versuchsreihen etwaige hierfür benötigte Temperatursensoren wieder entfernt werden, um Kosten zu sparen.

Das Verfahren gemäß Figur 3 kann auch vorsehen, eine Motortemperatur als Betriebsgröße zu erfassen, die von dem Temperatursensor 50 aus Figur 1 gemessen wird. Alternativ oder zusätzlich kann eine errechnete Motortemperatur als Betriebsgröße erfasst werden, wobei diese Motortemperatur in der nachstehend geschilderten Weise aus anderen Betriebsgrößen bestimmt wird. Die Motortemperatur kann aber ebenso dazu verwendet werden, um einen Betriebspunkt des Antriebs 30 zu definieren und diesem einen gemessenen Schmiermitteltemperaturwert zuzuordnen.

Bei der weiteren Variante gemäß Figur 4 wird die Schmiermitteltemperatur nicht aufgrund vorab hinterlegter Zusammenhänge oder Zuordnungen bestimmt, sondern aus den im laufenden Betrieb erfassten Betriebsgrößen errechnet. Hierfür wird in einem ersten Schritt S1 ein Erfassen der Betriebsgrößen ausgeführt, wobei es sich um dieselben Betriebsgrößen handeln kann, wie vorstehend aufgelistet. Insbesondere ist in diesem Fall vorgesehen, in einem nächsten Schritt S2 zunächst thermische Verlustleistungen innerhalb des Antriebs 30 zu bestimmen sowie im Schritt S3 darauf basierend eine Erwärmung von Bereichen, welche ein Schmiermittel enthalten, zu berechnen und daraus einen aktuell vorliegenden Schmiermitteltemperaturwert zu ermitteln. Anders ausgedrückt werden die thermischen Verlustleistungen als Wärmequellen innerhalb des Antriebs 30 betrachtet, welche zu einem Erwärmen des Schmiermittels in den verschiedenen Bereichen führen können.

Die einzelnen thermischen Verlustleistungen, die im Schritt S2 aus den erfassten Betriebsgrößen berechnet werden, beziehen sich zunächst auf thermische Verlustleistungen innerhalb des Traktionsmotors 1. Aus diesen Verlustleistungen kann eine Motortemperatur berechnet werden, wobei folgende Verlustleistungen zugrunde gelegt werden:

- Eisenverluste in den Blechpaketen des Ständers 22 und des Läufers 4. In der vorstehend allgemein geschilderten Weise kann hierfür auf die Betriebsgrößen der Stromstärke und Erregerfrequenz zurückgegriffen werden. Die Stromstärke kann zum Ermitteln eines magnetischen Flusses in den Blechpaketen verwendet werden, welche entsprechende Verluste verursachen. Die Erregerfrequenz kann auch mittelbar aus einer Drehzahl des Traktionsmotors 1 bestimmt werden.

- Wicklungsverluste bzw. Kupferverluste in den Wicklungen des Ständers 22 und des Läufers 4. Hierfür können in der vorstehend allgemein geschilderten Weise die der Stromstärke sowie einer Drehfeldleistung herangezogen werden, wobei letztere aus dem Betriebsgrößen der Drehzahl, der Stromstärke, der Spannung und der Zugkraft bestimmt werden kann.

- Reibungsverluste an Lagerstellen, insbesondere an den Lagerstellen 32 des Traktionsmotors 1. Hierfür wird eine Drehzahl des Traktionsmotors 1 berücksichtigt wie auch eine Lagerkraft, wobei sich letztere aus den Verzahnungskräften der Zahnräder des Getriebes 8 ergibt, welche sich an den Lagerstellen 32 abstützen.

Wie erwähnt, stellen die entsprechend berechneten Verlustleistungen jeweils Wärmequellen innerhalb des Traktionsmotors 1 dar. Der Traktionsmotor 1 aber auch die weiteren Komponenten des Antriebs 30, welche zumindest mittelbar wärmeleitend mit dem Traktionsmotor 1 verbunden sind, können daraufhin in an sich bekannter Weise mittels geeigneter Gleichungen thermodynamisch modelliert und/oder simuliert werden.

Beispielsweise kann unter Berücksichtigung von Wärmekapazitäten und einer Geometrie der einzelnen Bauteile sowie etwaigen Wärmeübertragungen in die Umgebung die Wärmeübertragung von dem Traktionsmotor 1 über die Wellen 18,19 in das Getriebe 8 und das dortige Ölbad berechnet werden. Hierfür können zum Beispiel zunächst die Wicklungsverluste am Rotor 4 sowie der Wärmeübergang von diesen Wicklungen auf die Läuferwelle 18 berechnet werden. In analoger Weise kann die Erwärmung der Lagerstellen 32 sowie die dortige Schmiermitteltemperatur berechnet werden, wobei diese Erwärmung wiederum ausgehend von den (inneren) thermischen Verlustleistungen des Traktionsmotors 1 bestimmt werden kann.

Die Gleichungen oder Gleichungssysteme, mit denen entsprechende Wärmeübertragungen berechnet werden, können allgemein analytisch und/oder numerisch gelöst werden. Hierfür kann auf auf dem Markt erhältliche Berechnungssoftware zurückgegriffen werden.

Als eine weitere Verlustleistung können Reibungsverluste in dem Getriebe 8 betrachtet werden. Wie vorstehend allgemein geschildert, können diese aufgrund der wirkenden Zahnkräfte und der Drehzahl bestimmt werden, wobei die Zahnkraft unter anderem aus der aufgebrachten Zugkraft sowie den Abmessungen bzw. der Geometrie des Getriebes 8 und dessen Zahnrädern berechnet werden kann. Derartige Reibungsverluste können wiederum Wärmequellen innerhalb des Getriebes 8 darstellen und sich zum Beispiel auf das darin befindliche Schmiermittel ausbreiten und/oder aufgrund von Wärmeübertragungen auch auf die Lagerstellen 32. Auch in diesem Fall können mittels einer herkömmlichen thermodynamischen Modellierung Gleichungen oder Gleichungssysteme aufgestellt werden, um Wärmeübertragung an und somit Temperaturen von einem Schmiermittel zu bestimmen, beispielsweise von dem Ölbad des Getriebes 8.

Prinzipiell ist es auch möglich, Reibungen in dem Gelenk 5 oder der Aufhängung 2 zu betrachten sowie damit einhergehende Verlustleistungen bzw. Erwärmungen zu berechnen. Hierzu kann wiederum auf vorliegende Betriebsgrößen zurückgegriffen werden, zum Beispiel in Form einer Drehzahl des Traktionsmotors 1, welche über die Läuferwelle in das Gelenk 5 übertragen wird.

Wurden auf diese Weise Temperaturwerte für Schmiermittel in interessierenden Bereichen bestimmt, kann wiederum für das dortige Schmiermittel der verbleibende und/oder verbrauchte Anteil der Gebrauchsdauer ermittelt werden (Schritt S4). Dies erfolgt wiederum gemäß den vorstehenden Ansätzen, wie insbesondere im Zusammenhang mit der ersten Variante von gemessenen Schmiermitteltemperaturen erläutert.

Als ein optionaler weiterer Schritt kann im Zusammenhang mit der Variante aus Figur 4 vorgesehen sein, die berechnete Motortemperatur mit einer von dem Temperatursensor 50 aus Figur 1 gemessenen Motortemperatur zu vergleichen. Prinzipiell ist es denkbar, auch die von dem Temperatursensor 50 gemessene Motortemperatur direkt zu verwenden, um Schmiermitteltemperaturen innerhalb des Traktionsmotors 1 zu bestimmen. Die hierfür zu berechnenden Wärmeübergänge können jedoch insbesondere im Betrieb des Antriebs 30 nur sehr schwer durch Gleichungen anzunähern sein und allgemein wenig präzise Ergebnisse liefern. Dennoch kann der Messwert des Temperatursensors 50 einen Indikator dafür liefern, inwiefern die anhand der Betriebsgrößen bzw. Verlustleistungen berechnete Motortemperaturen plausibel ist.

Insbesondere kann ein Korrekturfaktor eingeführt werden, mit dem die berechnete Motortemperatur korrigiert wird, falls diese ein vorbestimmtes Abweichkriterium von der gemessenen Motortemperatur erfüllt. Ein solches Kriterium kann zum Beispiel eine Abweichung von mehr als 20 % betreffen, woraufhin ein Korrekturfaktor basierend auf der festgestellten Abweichung eingeführt wird. Übergeordnet können somit die berechnete Temperaturen des Motors und auch der Schmierstoffe basierend auf der gemessenen Motortemperatur korrigiert werden, zum Beispiel in Form einer Art Driftkorrektur.

Ferner besteht bei der Variante aus Fig. 3 die Möglichkeit, im Rahmen einer Simulation und in Abhängigkeit von dabei vorliegenden (d.h. simulierten) Betriebsdaten verschiedene Betriebspunkte zu simulieren und hierfür jeweils eine Wert der Schmiermitteltemperatur in einem interessierenden Bereich zu berechnen. Somit können ähnlich wie bei dem Durchführen von Versuchsreihen Datensätze für einzelne Betriebspunkte hinterlegt werden. Wird dann im realen Betreib des Fahrzeugs aus den erfassten Betriebsdaten ermittelt, dass bzw. welcher Betriebspunkt vorliegt, können die vorab (d.h. während der Simulation) ermittelten Schmiermitteltemperaturen direkt aus dem entsprechenden Datensatz ausgelesen werden. Es muss sozusagen nicht fortlaufend im Betrieb berechnet werden, welche Temperaturen in Anbetracht aktueller Betriebsdaten gerade vorliegen.

Zusammengefasst können alternativ oder zusätzlich zu versuchstechnisch ermittelten Werten simulierte oder berechnete Temperaturwerte für ein etwaiges Abrufen oder Auslesen bei Vorliegen späterer realer Betriebspunkte vorab hinterlegt werden. Dies vermindert die im Betrieb erforderliche Rechenleistung und erhöht die Ermittlungsgenauigkeit.

Schließlich kann auch vorgesehen sein, dass im Rahmen derartiger Simulationen funktionale Zusammenhänge zwischen den Betriebsdaten (bzw. hierdurch definierten Betriebspunkten) und Schmiermitteltemperaturen in relevanten Bereichen ermittelt und hinterlegt werden. Ein solcher Zusammenhang kann vorzugsweise mittels nur einer Gleichung bzw. Funktion das Ermitteln einer Schmiermitteltemperatur in Abhängigkeit von Werten der Betriebsgrößen definieren. Im realen (Fahr-) Betrieb kann dann unter Umständen schneller und mit weniger Rechenleistung ein Schmiermitteltemperaturwert in Abhängigkeit aktuell vorliegender Betriebsdaten ermittelt werden, als wenn stets ein komplettes Gleichungssystem zu lösen wäre.

Ein gemeinsamer Vorteil der Varianten der Figuren 3 und 4 gegenüber einem tatsächlichen Messen von Schmiermitteltemperaturen ist, dass keine zusätzlichen Sensoren erforderlich sind. Stattdessen kann weitestgehend oder vollständig auf ohnehin bereits erfasste Betriebsdaten zurückgegriffen werden.

## Patentansprüche

1. Verfahren zum Verbessern des Schmiermitteleinsatzes in einem Antrieb (30) eines Fahrzeugs,

wobei der Antrieb (30) einen elektrischen Traktionsmotor (1) umfasst und wenigstens einen Bereich mit Schmiermittel, wobei das Verfahren umfasst:

- Ermitteln des Werts von wenigstens einer Betriebsgröße des Fahrzeugs;
- Ermitteln einer Schmiermitteltemperatur (T) basierend auf dem Wert der Betriebsgröße; und
- Abschätzen einer noch verfügbaren Gebrauchsdauer des Schmiermittels und/oder eines bereits verbrauchten Anteils der zulässigen Gebrauchsdauer des Schmiermittels basierend auf der Schmiermitteltemperatur (T);

wobei die Betriebsgröße wenigstens eine thermische Verlustleistung des Antriebs (30) ist.

2. Verfahren nach Anspruch 1,
wobei die Schmiermitteltemperatur (T) auch auf Basis eines gemessenen Temperaturwerts ermittelt wird, insbesondere wobei der Temperaturwert in dem oder nahe des Bereichs gemessen wird, in dem das Schmiermittel vorhanden ist.

3. Verfahren nach Anspruch 1 oder 2,
wobei für wenigstens eine weitere der folgenden Betriebsgrößen ein Wert ermittelt wird und die Schmiermitteltemperatur (T) auch basierend auf dem Wert dieser weiteren Betriebsgröße ermittelt wird:

- eine Zugkraft, die von dem Antrieb (30) aufgebracht wird;
- eine Drehzahl des Traktionsmotors (1);
- ein Drehmoment des Traktionsmotors (1);
- eine Fahrgeschwindigkeit des Fahrzeugs;
- eine Umgebungstemperatur;
- eine zum Antreiben des Traktionsmotors (1) verwendete Stromstärke;
- eine zum Antreiben des Traktionsmotors (1) verwendete Spannung;
- eine zum Antreiben des Traktionsmotors (1) verwendete Erregerfrequenz;
- eine Temperatur des Traktionsmotors (1).

4. Verfahren nach Anspruch 3,
wobei die Temperatur des Traktionsmotors (1) auf Basis von wenigstens einer der anderen Betriebsgrößen ermittelt wird.

5. Verfahren nach einem der vorangehenden Ansprüche,
wobei die thermische Verlustleistung eine der folgenden ist:

- eine Reibungsverlustleistung an wenigstens einer Lagerstelle (32) des Antriebs (30);
- eine Reibungsverlustleistung in einem Getriebe (8) des Antriebs (30);

- wenigstens eine Eisenverlustleistung des Traktionsmotors (1);
- wenigstens eine Wicklungsverlustleistung des Traktionsmotors (1).

**6.** Verfahren nach einem der vorangehenden Ansprüche,
wobei die Schmiermitteltemperatur (T) auf Basis eines hinterlegten Zusammenhangs zu Werten der Betriebsgröße ermittelt wird, insbesondere auf Basis eines hinterlegten tabellarischen Zusammenhangs.

**7.** Verfahren nach Anspruch 1,
wobei die Schmiermitteltemperatur (T) auf Basis des Werts der wenigstens einen Betriebsgröße berechnet wird.

**8.** Verfahren nach einem der vorangehenden Ansprüche,
wobei die Gebrauchsdauer auf Basis einer maximalen und/oder mittleren Schmiermitteltemperatur (T) über einen vorbestimmten Zeitraum abgeschätzt wird.

**9.** Antrieb (30) zum Antreiben eines Fahrzeugs, insbesondere eines Schienenfahrzeugs, mit:

- einem elektrischen Traktionsmotor (1);
- wenigstens einem Bereich mit einem Schmiermittel; und
- einer Ermittlungseinrichtung (60), die dazu eingerichtet ist, eine Schmiermitteltemperatur zu ermitteln, und darauf basierend eine noch verfügbare Gebrauchsdauer und/oder einen bereits verbrauchten Anteil der zulässigen Gebrauchsdauer des Schmiermittels abzuschätzen,
wobei die Ermittlungseinrichtung (60) ferner dazu eingerichtet ist, einen Wert von wenigstens einer Betriebsgröße des Fahrzeugs zu ermitteln sowie die Schmiermitteltemperatur (T) basierend auf dem Wert der Betriebsgröße zu ermitteln, wobei die Betriebsgröße wenigstens eine thermische Verlustleistung des Antriebs (30) ist.

**10.** Schienenfahrzeug,
umfassend einen Antrieb (30) nach Anspruch 9.

**Claims**

**1.** A method for improving usage of lubrication in a drive (30) of a vehicle,
wherein the drive (30) comprises an electric traction motor (1) and at least one region with lubricant,
wherein the method comprises the steps of:

- determining the value of at least one operating variable of the vehicle;
- determining the lubricant temperature (T) on the basis of the value of the operating variable; and
- estimating the remaining service life of the lubricant and/or the spent portion of the admissible service life of the lubricant on the basis of the lubricant temperature (T);
wherein the operating variable is at least one thermal dissipation of the drive (30).

**2.** The method according to claim 1,
wherein the lubricant temperature (T) is also determined on the basis of a measured temperature value, in particular wherein the temperature value is measured in or in the vicinity of the region in which the lubricant is provided.

**3.** The method according to claim 1 or 2,
wherein a value is determined for at least one further of the following operating variables, and the lubricant temperature (T) is also determined on the basis of the value of this further operating variable:

- a tractive force, which is generated by the drive (30);
- a speed of the traction motor (1);
- a torque of the traction motor (1);
- a speed of travel of the vehicle;
- an ambient temperature;
- a current used to drive the traction motor (1);
- a voltage used to drive the traction motor (1);
- an excitation frequency used to drive the traction motor (1);
- a temperature of the traction motor (1).

**4.** The method according to claim 3,
wherein the temperature of the traction motor (1) is determined on the basis of at least one of the other operating variables.

**5.** The method according to one of the preceding claims,
wherein the thermal dissipation is one of the following:

- a frictional dissipation at least at one bearing point (32) of the drive (30);
- a frictional dissipation in a transmission (8) of the drive (30);
- at least one core dissipation of the traction motor (1);
- at least one winding dissipation of the traction motor (1).

**6.** The method according to one of the preceding claims,
wherein the lubricant temperature (T) is determined on the basis of a stored relationship with values of the operating variable, in particular on the basis of a stored relationship in the form of a table.

7. The method according to claim 1,
wherein the lubricant temperature (T) is calculated on the basis of the value of the at least one operating variable.

8. The method according to one of the preceding claims,
wherein the service life is estimated on the basis of a maximum and/or mean lubricant temperature (T) over a predetermined period of time.

9. A drive (30) for driving a vehicle, in particular a rail vehicle, comprising:

- an electric traction motor (1);
- at least one region with a lubricant; and
- a determination device (60), which is designed to determine the lubricant temperature and, on this basis, to estimate the remaining service life and/or the spent portion of the admissible service life of the lubricant,
wherein the determination device (60) is also designed to determine a value of at least one operating variable of the vehicle and to determine the lubricant temperature (T) on the basis of the value of the operating variable, wherein the operating variable is at least one thermal dissipation of the drive (30).

10. A rail vehicle
comprising a drive (30) according to claim 9.

**Revendications**

1. Procédé pour améliorer l'utilisation de lubrifiant dans un entraînement (30) d'un véhicule,
dans lequel l'entraînement (30) comprend un moteur de traction (1) électrique et au moins une zone avec lubrifiant, dans lequel le procédé comprend :

- la détermination de la valeur d'au moins une grandeur de fonctionnement du véhicule ;
- la détermination d'une température de lubrifiant (T) sur la base de la valeur de la grandeur de fonctionnement ; et
- l'évaluation d'une durée d'utilisation restante du lubrifiant et/ou d'une proportion déjà utilisée de la durée de vie autorisée du lubrifiant sur la base de la température de lubrifiant (T) ;
dans lequel la grandeur de fonctionnement est au moins une puissance des pertes thermiques de l'entraînement (30).

2. Procédé selon la revendication 1,
dans lequel la température de lubrifiant (T) est déterminée également sur la base d'une valeur de température mesurée, en particulier dans lequel la valeur de température est mesurée dans ou à proximité de la zone, dans laquelle le lubrifiant est présent.

3. Procédé selon la revendication 1 ou 2,
dans lequel une valeur est déterminée pour au moins une autre des grandeurs de fonctionnement suivantes et la température de lubrifiant (T) est déterminée également sur la base de la valeur de ladite autre grandeur de fonctionnement :

- une force de traction, qui est appliquée par l'entraînement (30) ;
- une vitesse de rotation du moteur de traction (1) ;
- un couple de rotation du moteur de traction (1) ;
- une vitesse de déplacement du véhicule ;
- une température ambiante ;
- une intensité de courant utilisée pour entraîner le moteur de traction (1) ;
- une tension utilisée pour entraîner le moteur de traction (1) ;
- une fréquence d'excitation utilisée pour entraîner le moteur de traction (1) ;
- une température du moteur de traction (1).

4. Procédé selon la revendication 3,
dans lequel la température du moteur de traction (1) est déterminée sur la base d'au moins une des autres grandeurs de fonctionnement.

5. Procédé selon l'une quelconque des revendications précédentes,
dans lequel la puissance des pertes thermiques est une des suivantes :

- une puissance des pertes de frottement au niveau d'au moins un emplacement de support (32) de l'entraînement (30) ;
- une puissance des pertes de frottement dans une transmission (8) de l'entraînement (30) ;
- au moins une puissance des pertes dans le fer du moteur de traction (1) ;
- au moins une puissance des pertes d'enroulement du moteur de traction (1).

6. Procédé selon l'une quelconque des revendications précédentes,
dans lequel la température de lubrifiant (T) est déterminée sur la base d'un rapport enregistré par rapport à des valeurs de la grandeur de fonctionnement, en particulier sur la base d'un rapport enregistré sous forme de tableau.

7. Procédé selon la revendication 1,

dans lequel la température de lubrifiant (T) est calculée sur la base de la valeur de l'au moins une grandeur de fonctionnement.

8. Procédé selon l'une quelconque des revendications précédentes,
   dans lequel la durée de vie est évaluée sur la base d'une température de lubrifiant (T) maximale et/ou moyenne sur une période de temps prédéfinie.

9. Entraînement (30) pour entraîner un véhicule, en particulier un véhicule sur rails, avec :

   - un moteur de traction (1) électrique ;
   - au moins une zone avec un lubrifiant ; et
   - un dispositif de détermination (60) qui est mis au point pour déterminer une température de lubrifiant et pour évaluer sur cette base une durée de vie restante et/ou une fraction déjà utilisée de la durée de vie autorisée du lubrifiant, dans lequel le dispositif de détermination (60) est mis au point en outre pour déterminer une valeur d'au moins une grandeur de fonctionnement du véhicule ainsi que pour déterminer la température de lubrifiant (T) sur la base de la valeur de la grandeur de fonctionnement, dans lequel la grandeur de fonctionnement est au moins une puissance des pertes thermiques de l'entraînement (30).

10. Véhicule sur rails,
    comprenant un entraînement (30) selon la revendication 9.

Fig.1

Fig.2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015113306 A1
- DE 102007021524 A1
- US 2017138876 A1
- US 9739763 B2